# EUROPEAN PATENT APPLICATION

(11) **EP 0 792 586 A2**
(43) Date of publication of application: **03.09.1997**
(21) Application number: 97200479.0
(22) Date of filing: 19.02.1997
(51) Int. Cl.: A23L 1/03, A23L 1/30, A23D 7/00, A23L 1/24, C12N 1/20

(54) **Food products containing bacteria with cholesterol lowering activity**

(30) Priority: 28.02.1996 EP 96301336
(71) Applicant: UNILEVER N.V., NL-3000 DK Rotterdam (NL); UNILEVER PLC, London EC4P 4BQ (GB)
(72) Inventor: Lievense, Lourus Cornelis, Unilever, 3133 AT Vlaardingen (NL); Fletcher, John M.E., Sharnbrook, Bedfordshire, MK41 1LQ (GB); de Smet, Ingrid, University of gent, 9000 Gent (BE)
(74) Representative: Boerma, Caroline

(57) **Abstract**

The invention concerns bacteria having byle salt hydrolysis activity and optionally and preferably also bile salt polymerisation activity. Food products comprising such bacteria have been found to be capable to reduce the blood cholesterol level in the human blood. The effect is obtained by interference with the cholesterol and/or bile salt metabolism and is based on BSH activity of the bacteria. Food products which, on the basis of their normal daily intake provide a daily-BSH-intake of 0.3 micro-mol/min/kg bodyweight, are preferred, and even more preferred are food products providing at least a daily-BSH-intake of 0.6 micro-mol/min/kg bodyweight. In a further preferred embodiment a BSH activity of at least 0.8 micro-mol/min/1e10 cfu is obtained by the common, daily intake of food products comprising the bacteria. Further preferred are food products comprising fat, wherein at least 40% of the fat are poly unsaturated fatty acids.

## Description

One of the leading causes of death in the Western world today is heart disease. Numerous reports have provided evidence to link high blood cholesterol levels to coronary heart disease. For example, in one study (Lipids Research Clinics Program (1984) The lipid research clinics coronary primary prevention trial results. I Reduction in incidence of coronary heart diseases. J. Am. Med. Soc. 251: 351-363.), in which the influence of reducing serum cholesterol levels in reducing the risk of coronary heart disease was evaluated, the conclusion was drawn that reduction of high blood cholesterol levels significantly reduces the risk of heart attacks.
Cholesterol metabolism is closely linked with bile salt metabolism . Bile acids are synthesized in the liver from cholesterol and conjugated with glycine or taurine. Conjugated bile acids are transported to and stored in the gallbladder and are secreted when needed in the upper part of the small intestine (duodenum). Conjugated bile acids are essential in the emulsification and absorption of fats and lipids from the small intestine. They are reabsorbed in the lower part of the small intestine, to be transported by blood circulation to the liver. This constitutes the Entero-Hepatic Cycle (EHC) of bile salts.

During EHC, the bile salt pool (in total 5 to 10 mmol) is secreted several times a day (six on average) in the duodenum, and passes through the jejunum into the ileum (middle and lower part of the small intestine). During intestinal transit, the majority of the bile salts is reabsorbed to return to the liver via the portal vein. The daily faecal loss of bile salts that escape reabsorption is about 1 mmol. Since the body bile salt pool is approximately constant, this loss is to be newly synthesised from cholesterol.

Upon surgical, pharmacological or pathological interruption of the EHC of bile salts, bile salt synthesis is increased, leading to an increased demand for cholesterol in the liver. Apart from the therapeutical or surgical attempts to lower serum cholesterol levels through interruption of the EHC, it has been suggested that the ingestion of certain bacterial cells might also influence cholesterol levels through interference with bile salt metabolism.

Lactic acid bacteria (LAB) have been frequently associated with health-promoting effects in the human and animal intestinal tract, and the use of LAB as a probiotic has been a subject of interest for many years. One of the so-called probiotic effects of LAB is claimed to be the reduction of serum cholesterol levels. Although the underlying mechanism is not fully understood, it is suggested that the capacity to hydrolyse bile salts might be responsible for lowering the cholesterol level.

During intestinal transit, bile salts undergo a number of bacterial transformations, of which one of the most important is bile salt hydrolysis (BSH). The ability to hydrolyse bile salts is encountered in many intestinal bacteria, like *Lactobacillus* spp. *Enterococcus*, *Peptostreptococcu*s, *Bifidobacterium*, *Fusobacterium*, *Clostridium*, and *Bacteroides*. Upon bile salt hydrolysis, glycine or taurine is liberated from the steroid moiety of the molecule, resulting in the formation of free (deconjugated) bile salts.

Free bile salts are more easily precipitated at low pH or with Ca²⁺. They are less efficiently reabsorbed than their conjugated counterparts. Hence, they are more readily excreted in the faeces. Since the steady state requires that the amount of bile salts extracted from the EHC is matched by *de novo* synthesis of bile salts from cholesterol, elevated BSH activity will lead to an increased demand for cholesterol. Moreover, deconjugated bile salts might also physicochemically interact with cholesterol, i.e. co-precipitation of deconjungated bile salts and cholesterol at sufficiently low pH may occur (Klaver F.A.M. and Van der Meer R. (1993). The assumed assimilation of cholesterol by lactobacilli and *Bifidobacterium bifidum* is due to their bile salt deconjugating activity. Applied and Environmental Microbiology 59, 1120-1124.)

In conclusion it can be stated that transformation of bile salts by BSH active bacteria could lead to a decrease in blood cholesterol levels and thus a decrease in the change on coronary heart disease. Based on this hypothesis, several animal trials are described in literature, with inconclusive results. Reasons for prove of disprove of the above described hypothesis were numerous. Mainly the difference in bacterial strains, the origin of the strain (from which animal species it was derived), and the capacity to colonize the gut were mentioned. It is now our understanding that when above hypothesis was confirmed in animal trails, the poor control of food intake would be the reason for the observed decrease in blood cholesterol. Even small differences in the amount of food consumed can have large effects on blood cholesterol levels and this is particularly a problem in growing animals that have a large and variable appetite in relation to bodyweight.

In the present invention we have found that the main parameter that is of importance is the daily BSH activity intake (i.e. the product of the daily dosage and the BSH activity of the bacteria fed) per kg of bodyweight. Origin and colonization capacity is of minor importance. For the use in probiotic consumer goods, this new finding enables us to isolate BSH active bacteria from several sources, without being limited to bacteria stemming from human origin. The only additional issues that need to be provided are that the bacteria are food-grade and that a significant amount survives the passage through the stomach after consumption.

In order to obtain a significant blood cholesterol reduction from the probiotic food consumed, a sufficient daily intake of BSH activity per kg bodyweight must be assured. Since the required activity can be reached with large amounts of bacteria with relative low BSH activity as well as with relative low amounts of bacteria with high activity, the preferred daily dosage in this invention is expressed as the daily-BSH-intake. The BSH activity of bacteria is expressed as micro-mol of free bile salts formed per minute per 1e10 colony forming units (cfu) of viable bacterial cells (micro-mol/min/1e10cfu). Therefore, the preferred daily BSH intake is expressed as micro-mol/min/kg bodyweight. We have found that the minimum preferred daily-BSH-intake supplied by the probiotic food product is 0.3 micro-mol/min/kg of bodyweight. Assuming that at least 2%, preferably at least 20%, of the bacteria survive the passage through the stomach the preferred daily-BSH-intake provided by the probiotic food will be sufficient to show a significant reduction in blood cholesterol.

In prior art described wild type bacteria, selected in order to test the hypothesis described in Introduction, posses BSH-activities around 0.10 micro-mol/min/1e10 cfu. We have now found that these activities are not sufficient to reach the required daily-BSH-intake. For example, an average person of 70 kg would need to consume at least 2.1e12 cfu per day of these bacterial cells to reach the preferred daily-BSH-intake as described in this invention. It will not be possible to provide such amounts of bacteria in normal consumer food products for day to day use in a cost effective way. The bacteria we selected posses BSH-activities of at least 0.80 micro-mol/min/1e10cfu, preferably at least 1.50 micro-mol/min/1e10cfu, thereby reducing the amount of cells needed by a factor 15 (1.4e11cfu per day), which makes the application in consumer food products more feasible.

In another preferred embodiment of this invention it is claimed that intake of probiotic cells with BSH activity is accompanied by another activity, namely the bacterial activity to polymerise the deconjungated bile salts, which in turn are formed upon hydrolysis by BSH activity, for example polymerisation of deoxycholate to 3-alpha-poly-deoxycholate. Preferably these activities are combined in one bacterial strain, however, a probiotic food product consisting of one or more strains in that way providing sufficient levels of both activities will lower blood cholesterol as well.

The polymerised deconjungated bile salts formed by polymerisation activity of the bacterial cells will not be absorbed in the human small intestine, while deconjungated bile salts, formed by BSH-activity, are less readily absorbed than their tauro- or glyco-conjungated equivalents. That means that a combination of polymerisation activity and BSH activity will very effectively interfere with the cholesterol and/or bile salt metabolism in the human body. Therefore when these activities are supplied in combination in a probiotic food product, less daily-BSH-intake as such will be required The lower daily-BSH-intake will then be compensated by the presence of polymerisation activity which leads to the complete inhibition of the reabsorption of the formed deconjungated bile salts. In this invention, the polymerisation activity of bacterial cells is expressed as micro-mol of deconjungated bile salts polymerised per min per 1e10 cfu (micro-mol/min/1e10cfu). The claimed probiotic food product will therefore supply the sum of both activities at a minimum level of 0.3 micro-mol/min/kg bodyweight, while the ratio of BSH activity and polymerisation activity should preferably by greater than unity. Preferably, a minimum level of 0.6 micro-mol/min/kg bodyweight is supplied.

In another preferred embodiment of this invention, BSH-activity alone or together with polymerisation activity in probiotic food products is combined with other cholesterol lowering substances like polyunsaturated fatty acids as currently present in heart health fat based food products, providing that the food product supplies a minimum level of both activities as defined above.
In particular, fat containing food products of which the fat comprises at least 40% polyunsaturated fatty acids have been found to be very beneficial in this respect.

### EXAMPLES

### Example I

### HPLC determination of BSH-activity

Various bacteria were grown as a stirred culture on MRS (Difco) supplemented with CaCO₃ (1g/l) and anaerobic conditions (CO₂ on headspace). The final cultures were centrifuged (10000*g*) and concentrated to approximately 80 folds of the original broth volume. The cell pellet was mixed in equal amounts (w/w) with a cold solution of non fat dry milk (20% (w/w)) and stored at -30°C. After storage for one night the experiments were carried out with a fresh prepared solution of the frozen samples diluted in 10 mM sodium-acetate buffer (pH 5.6) to OD610 = 20 . To 0.5 ml of this solution, 0.5 ml 32 mM TCA-solution was added. The reaction tubes were placed in a waterbath at 37°C. After incubation for 5, 10, 15 or 30 minutes, the reaction was stopped by pasteurization of the reaction mixture (5 min, 90°C). After removing the biomass by centrifugation, the supernatant was filtered (0.8 µm) and stored at -30°C. The time-zero samples were prepared by pasteurizing the solution and additionally adding the substrate solution. Viable counts of the solution were performed on MRS agar plates after 10-fold dilution in peptone-physiological saline (1.0 g/l peptone, 8.5 g/l NaCl).

Taurocholic acid and cholic acid in the incubation mixture were separated by reversed-phase HPLC on a PLRP-S 100Å, 5µ, 150*4.6 mm column (Polymer Laboratories). The eluens was composed of 22 % acetonitrile in 0.1 M NaOH and it was used at a flow rate of 1.5 ml/min. The analytes were detected with a Decade pulsed amperometric detector (Antec-Leyden)equipped with a gold working electrode and an Ag/AgCl reference electrode. The pulse programme of the detector included three potentials: E1=0.03V with a duration time of 1.6 s (measuring potential), E2=0.6V with a duration time of 0.3 s (cleaning potential) and E3=-0.8V with a duration time of 0.3 sec (reduction of gold electrode surface). The column temperature was maintained at 35°C by a column thermostat. Samples were filtered through a 0.8 µm membrane filter and diluted until a concentration was obtained within the range of 0-15 µM. The injection volume was 50 µl. Standard solutions contained sodium cholate and sodium taurocholate respectively.

BSH activity was measured by the formed cholic acid and expressed as micro-mole/min/1e10 cfu.

### Example II

### Animal trial with pig as model system

Fourteen castrated male pigs, upon arrival weighing 15-20 kg, age 7-8 weeks were used. The pigs were weighed on arrival and at two week intervals throughout the experiment. Pigs were inspected daily for any evidence of diarrhoea, constipation or other illness and any observations recorded.

The experiment lasted for 12 weeks, during this time all pigs were fed the same, high cholesterol diet. At the end of the fifth week pigs were allocated to one of two groups in order to achieve approximately equal initial mean serum LDL and total cholesterol levels. For the next 4 weeks the test (T) group received strain RP32 (Gilliland S.E., Nelson C.R. and Maxwell C. (1985) Assimilation of cholesterol by *Lactobacillus acidophilus*. Appl. Env. Microbiol. 49 377-381) mixed in their feed and the control (C) group received an equivalent volume of bacterial growth medium (containing no bacteria) mixed in their feed. For the final 2 weeks of the experiment all pigs received the diet without additions. Faecal collections were obtained from each pig for a 24-hour period; before, during and after the 4 week period of RP32 feeding. A fresh faecal sample was taken from each pig before, during and after the period of RP32 feeding.

The diet was designed to have a similar composition as that described by Danielson A.D., Peo A.R., Shahani K.M., Lewis A.J., Whalen P.J.and Amer M.A. (1989) Anticholesterolemic property of *Lactobacillus acidophilus* yoghurt fed to mature boars. J. Anim. Sci. 67 966-974. The diet was analyzed for moisture, oil, protein, fibre and cholesterol . The cholesterol concentration of the diet was 0.23% (w/w). After arrival, pigs were acclimatized for two weeks. During the first week of the acclimatization period the feed was changed gradually from standard pig feed to the cholesterol containing experimental diet.

Strain RP32 was purchased from ATCC (American Type Culture Collection, Rockville, Maryland, USA) and stored at -80°C in 15% non-fat dry milk solution (NFDM). For the pig experiment, strain RP32 was grown as a stirred 14 L culture for approximately 16 hr at 34°C and pH 6.0 using MRS medium supplemented with CaCO₃ (1g/l). The final cultures were centrifuged (concentration was approx. 80 fold the original volume). The collected cell pellet was mixed in equal amounts (w/w) with a cold solution of NFDM (20% w/w) and stored at - 30°C. After 30 days of storage the suspension of cells showed no reduction in viability and no reduction in BSH-activity (0.13 micro-mol/min/1e10cfu).

Control material was prepared by using MRS medium in which glucose was replaced by lactic acid (20g/l) an adding CaCO₃ (1g/l). The thawed aliquots were added to the amount of water appropriate for each meal and mixed with feed. Aliquots of the control material were similarly dispensed.

Blood samples were obtained by puncture of the jugular vein. They were taken before the morning feed i.e. after an overnight fast. Serum was prepared and an aliquot of the serum was frozen and stored at -20°C for analysis of total cholesterol. A second aliquot was immediately processed for LDL analysis and the processed sample frozen at -20°C. Blood samples were taken on the sixth day of each week.

During the period of the experiment there were no significant differences in weight gain or feed intake between the control and probiotic-fed groups. The weight of pigs in both groups during probiotic feeding increased from 35 to 55 kg. Strain RP32 and its BSH activity was checked to be stable in the feed for at least 3 hours. On average each T pig received an RP32 dose of 7x10¹¹ cfu per day.

Serum total (3.4 mM) and LDL cholesterol (1.3 mM) concentrations remained relatively constant throughout the experiment for both groups. Similarly, at all sampling times there were no significant differences in the concentration of serum HDL cholesterol (1.8 mM) between control and RP32 fed pigs. In this well controlled animal trail, a cholesterol reduction with a daily-BSH-intake between 0.26 and 0.17 micro-mol/min/kg bodyweight (begin and end of probiotic feeding) could not be achieved.

### Example III

### Qualitative polymerised bile salt analysis

Faecal samples to be analyzed for polymerised bile salts were freeze-dried, pulverized and extracted during 30 minutes in a sonic water bath at 40°C using dichloromethane-methanol (1:1; v:v). The insoluble fraction was sedimented by centrifugation. The supernatant was removed to another tube and the extraction procedure was repeated. The collected supernatants were dried (nitrogen) and the residue was dissolved in a mixture (150 µl) of dichloromethane/methanol (1:1; v:v) and diluted with demineralized water to a final volume of 3 ml.

The sample was purified using a reversed solid phase extraction column (C18-cartridge) which was first washed with 3 ml of methanol followed by 3 ml water. The sample was then applied onto the column, the contents of which were then washed with 3 ml water, followed by two washes (2 x 3 ml) of a mixture of dichloromethane-methanol (1:1; v:v). The metabolites eluted by the two washes were separated by TLC. The reversed-phase TLC plates (RP-18, Merck) were pre-dried for 1 hour at 110°C. 10 µl of sample was applied and plates were developed in methanol-acetonitrile-water-formic acid 45:45:10:0.5 (v/v). After development, the eluent was evaporated and the plates were dried for 10 minutes at 110°C. To visualize free and polymerised bile acids, the plates were sprayed evenly with a mixture of methanol-water-sulphuric acid-MnCl₂.4H₂O (150 ml/150 ml/10 ml/1 g) and dried at 110°C for 15 minutes. Free and polymerised bile salts were visualized as fluorescent bands in UV light (254 nm). Polymerised bile salts remained at the bottom of the TLC plate.

### Example IV

### Animal trial with rat as model system

Twelve male rats (250g on average) were fed a semi-synthetic diet containing 0.1% cholesterol. Ten of the rats were ileostomized. After a recovery period of 14 days, fasted blood samples, ileal digesta samples and a 48 hr collection of excreted faeces were taken. Probiotic bacteria were then added to the diet daily. After 14 days of probiotic feeding, ileal digesta, a blood sample and a 48hr faecal collection were taken. For a further seven days rats were fed without addition of probiotic and ileal digesta, a blood sample and a 48hr faecal collection were again taken. The diet was then changed to contain 1.0% cholesterol and the sampling procedures for ileal digesta, blood and faecal collections were repeated before, during and after a 14 day period of probiotic feeding. The diets were mixed with deionized water (in the ratio; 1.5/1, water/diet) just before feeding.

The bacteria used in this experiment is a *Lactobacillus animalis* (strain 364) isolated from hamster faeces. Strain 364 was identified as a *L.animalis* strain by SDS-page analysis. Strain 364 has a very high BSH activity (0.8 micro-mol/min/1e10cfu), compared with other intestinal strains, and was additionally sub-selected to possess resistance to streptomycin. The bacteria were grown as a stirred 10L and free acidifying culture for approximately 16hr at 34°C using MRS-medium supplemented with CaCO₃ (1g/l). For preparation of the feeding samples, the collected cell pellet (80 times concentrated) was mixed in equal amounts (w/w) with a cold solution consisting of 20% (w/w) non fat dry milk and stored at -30°C.

The bacteria were given as an addition to the food of the rats. Because the bacteria are prepared in an aqueous suspension it was necessary to feed the diet mixed with water. The bacteria were supplied frozen in vials sufficient for feeding all the rats at each meal. The viability and activity of the bacteria after thawing and the stability of the bacteria in their BSH-activity in the diet was controlled and found stable.

The amount of food required per meal for the group of rats was calculated from the food consumption data obtained in the first 7 days of the study, plus 10% for spillage and wastage. The amount of bacteria per vial, to be mixed with food and water, was calculated to provide 10¹¹ live bacteria per day.

Blood samples were taken before the morning feeding from the cut tip of the tail. Serum was prepared and total cholesterol measured. The ileal samples were taken from anaesthetized rats at approximately 12.00 hr before, during and after probiotic feeding.

There was no difference in growth rate and food intake between periods of control and probiotic feeding. There was no incidence of ill-health associated with probiotic feeding. The number of lactobacilli found in ileal digesta when grown on MRS agar, with and without streptomycin were analyses. There was a small increase in total lactobacilli during feeding of strain 364 on both low and high cholesterol diets. There was however a much larger increase in streptomycin resistant lactobacilli during feeding of strain 364. It was calculated that more than 20% of the bacteria fed survived the passage through the stomach.

When fed the low cholesterol diet there was approximately a 6% reduction in blood cholesterol levels during bacteria feeding (from 2.45 to 2.30 mM) and when fed the high cholesterol diet there was approximately a 7% reduction (from 2.78 to 2.58 mM). This cholesterol reduction in rat was achieved with a daily-BSH-intake of 32 micro-mol/min/kg bodyweight. Polymerised bile salts were found in higher amounts in the faeces of the rats in the probiotic feeding group than in the control group, as visually and qualitatively determined with the procedure as described above.

### Example V

### Animal trial with pigs as model system

Twenty pigs (10 females and 10 castrated males) of about 30 kg live weight at the start of the experiment, age 10 weeks, were divided into two experimental groups, the control pigs and the pigs to be treated with the probiotic supplement. The allocation of the pigs to one of the groups was performed in such a way that both groups showed equal distributions of the sexes, equal initial weights and cholesterol levels. The pigs were inspected daily for any evidence of diarrhoea, constipation or other illnesses or observations. The pigs were weighed at the beginning and end of each experimental period.

The experiment lasted for 13 weeks. During the first five weeks, all pigs received a diet rich in saturated fat and low in fibre content, which contained 0.2% (w/w) cholesterol. During the following five weeks, the animals were fed the same diet in which the cholesterol content was doubled (0.4% w/w). This high-fat diet was supposed to initially lead to increased serum cholesterol levels. During probiotic feeding, from week 4 up to and including week 7, the treated group received the probiotic strain both in the morning and evening feed. During the last three weeks, all animals received a regular diet without cholesterol addition.

The *Lactobacillus* strain used was isolated from fresh pig faecal material on Rogosa agar plates (Oxoid). The strain was identified as a *L. reuteri* strain by SDS-page analysis. Its BSH activity was characterised as 2.54 micro-mol/min/1e10 cfu. The strain was fermented during 36 h at 37 °C in MRS broth supplemented with 1.0 g/l CaCO₃. The final fermentation cultures were centrifuged. The pellet was harvested and dissolved in spent medium supernatant to obtain a 50-fold concentration of the original fermentation volume. Subsequently, this was mixed in equal amounts with a cold solution of [30% (v/v) glycerol and 70% (v/v) non-fat dry milk (20%; w/v)] and stored at -70 °C in appropriate aliquots. Prior to feeding, the probiotic supplements were then thawed at room temperature and added to the water to be mixed with the feed. Viable cell counts of the stored cultures were regularly checked. A dose of 1e11 cfu of the strain was added to both the morning and afternoon feed of the treated pigs during the probiotic feeding.

The daily feed intake of the pigs was carefully controlled because, firstly, blood cholesterol levels are very sensitive to the amount of food consumed, and secondly, because the strain was mixed with the feed. Therefore, the amount of feed, which was administered in two equal meals at 8.00 h and 16.00 h respectively, was optimized/restricted to assure complete intake. During the probiotic treatment the pigs increased in weight from 43 to 66 kg.

Blood samples were taken by venepuncture at the beginning and end of each experimental period, including an extra sampling after two weeks of *L. reuteri* dosage. The samples were taken after an overnight fast, *i.e.* before the morning feeding at 8.00 h. Serum was prepared and immediately analyses for total cholesterol, HDL-cholesterol and triglycerides content. LDL-cholesterol was calculated from the difference between total and HDL-cholesterol and triglycerides

After two weeks of *Lactobacillus* feeding (low cholesterol diet), the total and LDL-cholesterol levels in the treated pigs were reduced by 11 respectively 26% compared to the control animals, while after four weeks (high cholesterol diet), these reduction levels were 15 and 24 % respectively. When comparing the evolution in time of the total serum cholesterol, it was clear that the total cholesterol levels in the treated pigs were lowered during the four weeks probiotic feeding and increased during the three weeks post-treatment follow-up. In the control pigs, a gradual increase of the total blood cholesterol concentration was observed for that seven weeks period. This evolution was also observed for LDL-cholesterol, while no consistent changes were found for the HDL-cholesterol levels. The average increase of total cholesterol levels during the ten weeks high-fat, high-cholesterol feeding for the control and treated pigs were about 35% and 15% respectively, whereas the LDL-cholesterol levels showed average increases of about 75% and 35% for the control and treated animals respectively. This clearly demonstrates the cholesterol controlling effect of the *L. reuteri* cells when fed at an daily-BSH-intake between 1.18 and 0.77 micro-mol/min/kg bodyweight (begin and end of probiotic feeding).

Compared to the initial value, the faecal bile salt excretion of the control pigs was increased with 13% at week 6, 26% at week 8, and 25% at week 10. This clearly demonstrates the effect of diets rich in fat and cholesterol on faecal bile salt concentrations. Nevertheless, the total faecal bile salt excretion in the treated pigs was about 25% higher compared to the control pigs after one week of *L. reuteri* ingestion. This higher output lasted until the end of the *Lactobacillus* treatment. After the treatment was stopped, this level decreased to the level of the control animals. Polymerised bile salts were found in higher amounts in the faeces of the pigs in the probiotic feeding group than in the control group, as visually and qualitatively determined with the procedure as described above.

### Example VI

### Preparation of a spread

87 parts refined sunflower oil (65% PUFA as linoleic acid) and 13 parts of a refined interesterified mixture of 50 parts fully hardened palm oil and 50 parts fully hardened palm kernel oil are mixed. To 70 parts of this fatblend, 0.1 part soybean lecithin, 0.1 part monoglyceride and a small amount of β-carotene solution are added.

To 26 parts water, 0.5 part whey protein powder, 0.1 part salt, a small amount of flavour, and citric acid to obtain a pH of 4.6 are added. To this water phase, 3 parts of a thawed concentrated solution of *L. reuteri* strain (as described in one of the previous examples), containing 2.1e11 cfu/g in a protecting mixture of glycerol and non-fat dry milk, is added.

70 parts of the fat phase composition (kept at 50°C) and 30 parts of the aqueous phase composition (kept at 20°C) are mixed using a proportioning system. The mixture is then passed through a Votator line with 2 scraped surface heat exchangers (A-units) and 1 stirred crystallizer (C-unit) operating at 100 rpm. The product leaving the C-unit has a temperature of 11°C. It is filled into tubs and stored at 5°C. A good fat continuous spread is obtained. It contains 57% PUFA on fat blend and 6.3e9 cfu/g product of *L.reuteri.*

### Example VII

### Preparation of a spread

A *Bifidobacterium infantis* strain was fermented during 45 h at 37 °C in MRS broth supplemented with 0.05% cystein-HCL under anaerobic conditions. The final fermentation culture was centrifuged. The pellet was harvested and dissolved in a 20% non fat dry milk solution to obtain a 100-fold concentration of the original fermentation volume. This concentrate contained 4e11 cfu/g of the *B.infantis* strain. The BSH activity of *B.infantis* was characterised as 1.79 micro-mol/min/1e10 cfu.

To obtain a spread, a similar procedure as described in previous example was followed. However, to 27 parts of water, 2 parts of the *B.infantis* concentrate was added. Other processing was similair than in previous example. A good fat continuous spread was obtained. It contains 57% PUFA on fat blend and 8e9 cfu/g product of *B.infantis*. The viable count remained stable during storage at 5°C for at least 10 weeks.

### Example VIII

### Preparation of a dressing

15 parts of pasteurized drink yoghurt is mixed with 2 parts of acidic acid (10%), 10 parts of sugar, 5 parts of *B.infantis* concentrate as described above, and 43 parts of water. To this mixture 10 parts of various flavour components, preservatives, thickeners and emulsifiers are added. The mixture is thouroughly mixed in a stainless steel stirred vessel. To this aquous mixture 15 parts of sunflower oil is added, thourouhly mixed for an additional 15 min, to obtain a pre-emulsion. The pre-emulsion is brought into a colloid mill (Prestomill PM30) and processed at a split-size between level 15 and 20 and a throughput between level 4 and 6. A good water continuous dressing was obtained. It contains 2e10 cfu/g product of *B.infantis*. The viable count remained stable during storage at 5°C for at least 7 weeks.

## Claims

1. Food products which lower blood cholesterol based on the presence of bacteria which after gastric transit will interfere with the cholesterol and/or bile salt metabolism and were the interference with cholesterol and/or bile salt metabolism is based on bile salt hydrolysis activity of the bacteria.

2. Food products according to claim 1 which provide at least a daily-bile salt hydrolysis-intake of 0.3 micro-mol/min/kg bodyweight.

3. Food products according to claim 2 which provide at least a daily-bile salt hydrolysis-intake of 0.6 micro-mol/min/kg bodyweight.

4. Food products according to claim 3, which contain bacteria with a bile salt hydrolysis activity of at least 0.8 micro-mol/min/1e10 cfu.

5. Food products according to claim 1, where the interference with cholesterol and/or bile salt metabolism is additionally based on free bile salt polymerisation activity of foodgrade bacteria.

6. Food products according to claim 5, which provide at least a daily combined bile salt hydrolysis and free bile salt polymerisation activity of 0.3 micro-mol/min/kg bodyweight.

7. Bacteria selected on their bile salt hydrolysis activity, with bile salt hydrolysis activities sufficient enough to be used in foods in order to interfere with the cholesterol and/or bile salt metabolism in the human body.

8. Bacteria according to claim 7 with a bile salt hydrolysis activity of at least 0.8 micro-mol/min/1e10cfu.

9. Bacteria according to claim 7 with a combined bile salt hydrolysis and bile-salt-polymerisation activity.

10. Fat containing food products according to any one of claims 1 to 6, wherein the fat in the product comprises at least 40% of poly-unsaturated fatty acids.
